# NEUE EUROPÄISCHE PATENTSCHRIFT

(11) **EP 2 639 292 B2**
(45) Veröffentlichungstag und Bekanntmachung des Hinweises auf die Entscheidung über den Einspruch: **25.11.2020**
(45) Hinweis auf die Patenterteilung: 29.11.2017
(21) Anmeldenummer: 13159032.5
(22) Anmeldetag: 13.03.2013
(51) Int. Cl.: C12M 1/34, G01N 15/02, G01N 21/25, G01N 21/59, G01N 21/64, G01N 21/76, G01N 33/543, G01N 35/02, G06K 9/00, G06T 7/00, G01N 15/14, G01N 33/50

(54) **Verfahren und Mikroplatten-Reader zum Untersuchen von biologischen Zellen oder Zellkulturen**
Method and micro-plate reader for examining biological cells or cell cultures
Procédé et lecteur de microplaquettes pour l'analyse de cellules ou de cultures cellulaires biologiques

(30) Priorität: 14.03.2012 CH 3652012; 14.03.2012 US 201261610647 P
(43) Veröffentlichungstag der Anmeldung: 18.09.2013
(73) Patentinhaber: Tecan Trading AG, 8708 Männedorf (CH)
(72) Erfinder: Gebetsroither, Harald, 5082 Grödig (AT); Gfrörer, Andreas, 82549 Königsdorf (DE); Koota, Juha, 83471 Berchtesgaden (DE)
(74) Vertreter: Troesch Scheidegger Werner AG

(56) Entgegenhaltungen:
- DE-A1- 19 916 748
- US-A1- 2003 103 662
- US-A1- 2003 127 609
- US-A1- 2005 051 723
- US-A1- 2005 213 374
- US-A1- 2006 094 868
- US-A1- 2007 177 149
- RAVI KAPUR ET AL: 'STREAMLINING THE DRUG DISCOVERY PROCESS BY INTEGRATING MINIATURIZATION, HIGH THROUGHPUT SCREENING, HIGH CONTENT SCREENING, AND AUTOMATION ON THE CELLCHIP TM SYSTEM' BIOMEDICAL MICRODEVICES, KLUWER, DORDRECHT, NL Bd. 2, Nr. 2, 01 Januar 1999, Seiten 99 - 109, XP002162337 DOI: 10.1023/A:1009993519771 ISSN: 1387-2176
- Anonymous: "Lichtquelle", Wikipedia Wikipedia, Wikipedia, 18 May 2018 (2018-05-18), XP055547932, [retrieved on 2019-01-28]

## Beschreibung

Die Erfindung betrifft ein Verfahren zum Untersuchen von biologischen Zellen oder Zellkulturen in einem Mikroplatten-Reader. Dieses Verfahren umfasst das Aufnehmen von zumindest einer Mikroplatte mit biologischen Zellen enthaltenden Wells mit einer Aufnahmeeinrichtung des Mikroplatten-Readers; das Positionieren der Aufnahmeeinrichtung mit den biologischen Zellen enthaltenden Wells der Mikroplatte(n) gegenüber Messeinrichtungen des Mikroplatten-Readers und das Erfassen zumindest eines integralen Signals (z.B. der Lumineszenz aller Proben in einem Well einer Mikroplatte) mit zumindest einer der Messeinrichtungen.

Zudem ist das Positionieren der Aufnahmeeinrichtung mit den biologischen Zellen enthaltenden Wells der Mikroplatte(n) gegenüber Aktionsquellen des Mikroplatten-Readers; das Herbeiführen einer Wechselwirkung zwischen zumindest einer dieser Aktionsquellen und biologischen Zellen in bestimmten Wells der Mikroplatte(n) mit zumindest einer der Aktionsquellen zum Hervorrufen oder Erzeugen eines messbaren Signals; und das Erfassen zumindest eines integralen Signals (z.B. der Fluoreszenz oder Absorbance aller Proben in einem Well einer Mikroplatte), das durch die Aktionsquelle(n) in oder an biologischen Zellen in den bestimmten Wells der Mikroplatte(n) hervorgerufen oder erzeugt wurde, vorgesehen. Die Messung der Fluoreszenz von Zellen oder deren Stoffwechselprodukten ist aus dem Stand der Technik bekannt. So offenbart EP 2 253 983 A2 ein Rastermikroskop zur spektralen Erfassung der emittierten Fluoreszenz von Proben, die mit einem Laser angeregt und einem Streugitter in unterschiedliche Wellenlängenbereiche aufgeteilt und einer Multi-Anode-Photomultipier-Röhre zugeführt wird. Das Dokument ITMI912510 A1 offenbart einen sogenannten Mikroplatten-Reader, mit welchem Proben in Wells von Mikroplatten mit polarisiertem Licht bestrahlt und das dabei ausgelöste ebenfalls polarisierte Fluoreszenzlicht mit einer Photomultipier-Röhre detektiert wird. Mikroplatten-Reader die mit nicht-polarisiertem Anregungslicht arbeiten, sind ebenfalls seit langem bekannt.

Es sind auch Dokumente bekannt (z.B. JP 58 062 542 A oder JP 11 037 923 A), die das Abbilden von Kohäsionsmustern von Partikeln oder biologischen Zellen auf dem Boden der Wells von Mikroplatten mit einer Digitalkamera offenbaren, welche einen Bildgebenden CCD- (= Charge Coupled Device) oder CMOS- (= Complementary Metal Oxide Semiconductor) Sensor umfasst.

Das Erfassen der Lumineszenz von Zellen in Wells von Mikroplatten ist beispielsweise aus DE 102 36 029 A1 bekannt. Offenbart wird ein Dispenser mit welchem durch Zugeben von Flüssigkeiten zu den Proben in den Wells einer Mikroplatte Lumineszenz ausgelöst wird und eine CCD-Kamera, mit der die durch die durchsichtigen Böden der Mikroplattenwells austretende Lumineszenz über ein optisches System fotografiert wird.

Aus WO 2006/031537 A2 ist ein Verfahren und eine Vorrichtung zum Untersuchen von biologischen Zellen in Mikroplatten bekannt. Die Mikroplatte wird in einem Zielgebiet beleuchtet und das von den Zielgebieten stammende Licht wird zu einem Arraydetektor geführt, der diskrete Ziele innerhalb der Mikroplattenwells im Zielgebiet als Teilbilder detektiert und der die Teilbilder zu einem Gesamtbild des Mikroplattenwells zusammensetzt. Dabei können zelluläre Mikroarrays oder auch ungeordnete biologische Zellen erfasst werden.

Das Zählen von biologischen Zellen in speziellen als Zählkammern ausgebildeten Objektträgern ist seit langem aus dem Gebiet des Zählens von Blutzellen mit dem Lichtmikroskop bekannt. Automatisierte Zell-Zählsysteme oder "Cell Counter" unterschiedlicher Hersteller sind ebenfalls bekannt. Diese Cell Counter zeichnen sich insbesondere dadurch aus, dass eine Zählkammer mit biologischen Zellen in das Zählsystem eingeschoben werden kann, worauf das Zählsystem selbständig die Zähloptik fokussiert, die biologischen Zellen zählt und das Zählergebnis anzeigt.

Typischerweise werden in Labors, die Untersuchungen an biologischen Zellen bzw. Zellkulturen in Mikroplatten durchführen, eine mit Licht oder elektrisch induzierte Lumineszenz erzeugt und diese Lumineszenz mit einer Photomultiplier-Röhre oder einem Halbleiter-Photomultiplier gemessen oder mit einer Bildgebenden Kamera abgebildet. Zum Interpretieren der Messresultate bzw. der Bilder wird die Mikroplatte mit einem Lichtmikroskop untersucht und es wird festgestellt, ob sich die biologischen Zellen noch in einen normalen physiologischen Zustand befinden, oder ob sie eine abnorme Form aufweisen oder gar verendet sind.
Es ist auch ein Zellmessgerät auf dem Markt (IsoCyte Laser Scanning Cytometer, Molecular Devices, Sunnyvale, California, USA), das die biologischen Zellen in den Wells von Mikroplatten mit einem Laserstrahl abrastert und gleichzeitig vom Laserstrahl erzeugte Fluoreszenz- oder Streu-Signale mit einem oder mehreren Photomultipliern erfasst, die in einem Well vorhandenen biologischen Zellen identifiziert und zählt und ein Bild des abgerasterten Wells produziert. Mittels Bildverarbeitung können die einzelnen Zellen in jedem Bild identifiziert und deren Aktivität aufgelistet werden. Die Resultate aller Zellen eines Wells können integriert werden.
Es ist die Aufgabe der vorliegenden Erfindung ein alternatives Verfahren zum Untersuchen von biologischen Zellen oder Zellkulturen in Mikroplatten und eine zum Durchführen des Verfahrens geeignete Vorrichtung vorzuschlagen.
Diese Aufgabe wird gemäss einem ersten Aspekt mit den Merkmalen des unabhängigen Anspruchs 1 anhand eines Verfahrens zum Untersuchen von biologischen Zellen oder Zellkulturen in einem Mikroplatten-Reader gelöst.

Diese Aufgabe wird gemäss einem zweiten Aspekt mit den Merkmalen des unabhängigen Anspruchs 13 anhand eines Mikroplatten-Readers zur Verwendung im erfindungsgemässen Verfahren zum Untersuchen von biologischen Zellen oder Zellkulturen gelöst.

Zusätzliche Weiterbildungen des erfindungsgemässen Verfahrens bzw. des erfindungsgemässen Mikroplatten-Readers und weitere erfindungsgemässe Merkmale ergeben sich aus den abhängigen Ansprüchen.

Vorteile des erfindungsgemässen Verfahrens bzw. der erfindungsgemässen Vorrichtung umfassen:
- Zur Kombination von quantitativen Messungen von Zellparametern in Mikroplatten-Readern mit mikroskopischen Bildern zur qualitativen Inspektion des Status dieser Zellkulturen wurden bisher zwei unterschiedliche Geräte benötigt, ein Mikroplatten-Reader und ein Bildgebendes Mikroskop. Die vorliegende Erfindung ermöglicht nun das Durchführen dieser beiden ergänzenden Untersuchungen in ein und demselben Gerät.
- Bisher mussten die Mikroplatten mit den Zellkulturen aus dem Mikroplatten-Reader genommen und zum Mikroskop transferiert werden. Dieser umständliche Schritt musste von einer Bedienungsperson ausgeführt werden und bedingte zudem eine gewisse Zeitverzögerung zwischen den Untersuchungsmethoden. Die vorliegende Erfindung ermöglicht nun das Automatisieren der Kombination dieser beiden ergänzenden Untersuchungen in einem einzigen Gerät und mit minimalsten Zeitdifferenzen zwischen dem Erfassen eines integralen Signals in einem bestimmten Mikroplattenwell und dem Abbilden dieses Mikroplattenwells.
- Zum Transferieren der Mikroplatten von einem Mikroplatten-Reader zu einem Mikroskop wurden bisher die empfindlichen Zellkulturen oft den herrschenden Umweltbedingungen ausgesetzt oder mussten umständlich gegen diese abgeschirmt werden. Die vorliegende Erfindung ermöglicht vorzugsweise das Aufrechterhalten einer kontrollierten Atmosphäre in den Mikroplattenwells mit den Zellkulturen während dem Durchführen aller quantitativen und qualitativen Untersuchungen an diesen biologischen Zellen.
- Im erfindungsgemässen Mikroplatten-Reader, der sich zur Durchführung des erfindungsgemässen Verfahrens eignet, ist ein Modul mit Bildgebenden Eigenschaften integriert, so dass biologische Zellen und Zellkulturen, die sich beispielsweise auf dem Boden der Mikroplattenwells befinden, mit mikroskopischer Auflösung visualisiert werden können.
- In einer bevorzugten Ausführungsform ist der erfindungsgemässe Mikroplatten-Reader als Multi-Mode-Reader ausgebildet und erlaubt eine praktische beliebige und bevorzugt automatisierte Kombination der Erfassens von integralen Signalen, wie Fluoreszenz, Lumineszenz oder Absorbance in ausgewählten Mikroplattenwells mit dem mikroskopischen Abbilden dieser Mikroplattenwells.
- Die integralen Signale, wie Fluoreszenz, Lumineszenz oder Absorbance dienen der Messung von Zellaktivitäten. Signalintensitätsänderungen hängen ab sowohl von Änderungen der Zellaktivität als auch von der Änderung der Zellzahl (z. B. durch Zellvermehrung).
- Das Feststellen der Konfluenz der Zellen oder Zellkulturen auf dem Boden eines Mikroplattenwells gibt das Verhältnis der von einer Zellkultur bedeckten Bodenfläche zur noch freien Bodenfläche dieses Mikroplattenwells wieder. Dieses Verhältnis erlaubt für Zellkulturen mit adhärenten Zellen wiederum die Normierung des integralen Signals mit dem Zellwachstum und somit die unverfälschte, automatisierte Erfassung der Zellaktivität.

Das erfindungsgemässe Verfahren zum Untersuchen von biologischen Zellen oder Zellkulturen in einem Mikroplatten-Reader und der entsprechende Mikroplatten-Reader werden nun an Hand von schematischen Zeichnungen, die ausgewählte beispielhafte Ausführungsformen darstellen und den Umfang der Erfindung nicht einschränken sollen, näher erläutert. Dabei zeigt:
- Fig. 1: einen Vertikalschnitt durch einen Mikroplatten-Reader gemäss einer ersten nicht erfindungsgemässen Ausführungsform beim Einschieben einer Mikroplatte in den vorzugsweise als lichtdicht abschliessbare Kammer ausgebildeten Probenraum;
- Fig. 2: einen Vertikalschnitt durch einen Mikroplatten-Reader gemäss einer zweiten Ausführungsform beim Erfassen von integralen Signalen in ausgewählten Mikroplattenwells bzw. beim mikroskopischen Abbilden von Mikroplattenwells in einem vorzugsweise als lichtdicht und gasdicht abschliessbare Isolierkammer ausgebildeten Probenraum;
- Fig. 3: einen Vertikalschnitt durch einen Mikroplatten-Reader gemäss einer dritten Ausführungsform beim Erfassen von integralen Signalen in ausgewählten Mikroplattenwells in einem vorzugsweise als lichtdicht und gasdicht abschliessbare Isolierkammer ausgebildeten Probenraum;
- Fig. 4: eine Aufsicht auf Mikroplattenwells mit auf dem Wellboden aufgebrachten, beispielhaften Elektroden und mit auf den Wellwänden aufgebrachten, beispielhaften elektrischen Kontakten;
- Fig. 5: eine schematische Darstellung des Strahlengangs der Durchlicht-Beleuchtung durch ein Well einer Mikroplatte, wobei:
Fig. 5A eine konventionelle Durchlicht-Beleuchtung mit eingezeichnetem Verlauf eines transmittierten Lichtstrahlbündels,
Fig. 5B eine erfindungsgemässe Durchlicht-Beleuchtung mit eingezeichnetem Verlauf eines transmittierten Lichtstrahlbündels,
Fig. 5C eine konventionelle Durchlicht-Beleuchtung mit eingezeichnetem Verlauf eines transmittierten Lichtstrahls, und
Fig. 5D eine erfindungsgemässe Durchlicht-Beleuchtung mit eingezeichnetem Verlauf eines transmittierten Lichtstrahls zeigt;
- Fig. 6: Abbildungen eines Wells in Durchlicht-Beleuchtung zeigt; wobei:
Fig. 6A das Resultat einer konventionellen Beleuchtung, und
Fig. 6B das Resultat einer erfindungsgemässen Beleuchtung zeigt;
- Fig. 7: Intensitätsverteilungen in den Abbildungen von Fig. 6 über einen ganzen Durchmesser der Wells; wobei:
Fig. 7A die Intensitätsverteilung bei konventionellen Beleuchtung, und
Fig. 7B die Intensitätsverteilung bei erfindungsgemässer Beleuchtung zeigt;
- Fig. 8: den Vergleich der beiden Abbildungen von Fig. 7A und 7B nach einer jeweiligen Normierung der Belichtungszeit.

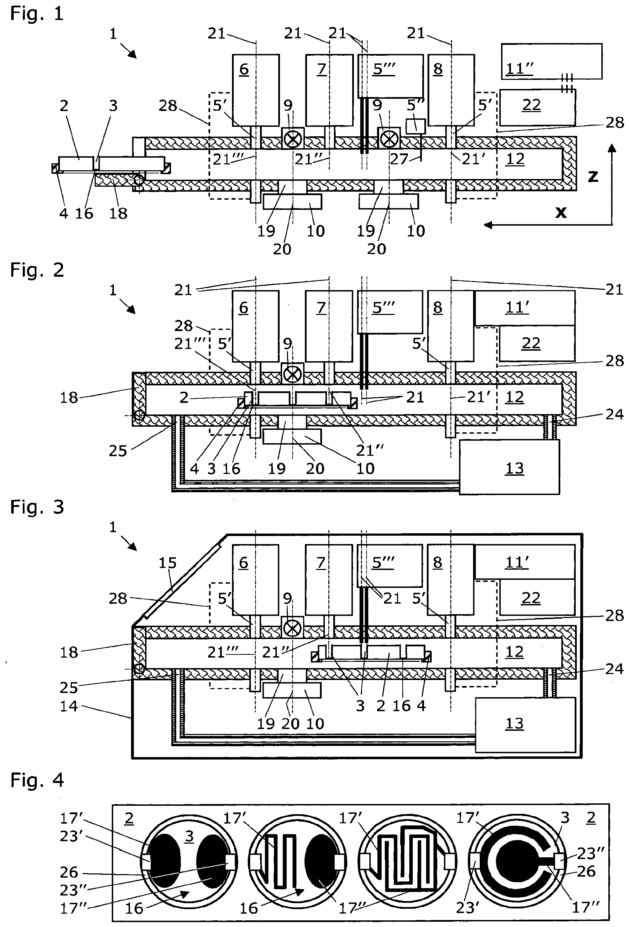

Die Figur 1 zeigt einen Vertikalschnitt durch einen Mikroplatten-Reader 1 beim Einschieben einer Mikroplatte 2 in den vorzugsweise als lichtdicht abschliessbare Kammer ausgebildeten Probenraum 12. Dieser Mikroplatten-Reader 1 umfasst eine Aufnahmeeinrichtung 4 zum Aufnehmen von zumindest einer Mikroplatte 2 mit biologischen Zellen oder Zellkulturen enthaltenden Wells 3. Diese Aufnahmeeinrichtung 4 ist vorzugsweise so weit aus dem Probenraum 12 des Mikroplatten-Readers ausfahrbar ausgebildet, dass zumindest eine Mikroplatte 2 von Hand oder mittels eines Mikroplatten-Handling-Roboters (beides nicht dargstellt) in diese Aufnahmeeinrichtung 4 eingelegt bzw. davon abgehoben werden kann. Die Aufnahmeeinrichtung 4 ist hier bereits teilweise eingefahren, weil gerade eine Mikroplatte 2 in den Mikroplatten-Reader 1 eingeschoben wird. Während des Einschiebens oder Auswerfens einer Mikroplatte ist vorzugsweise eine Klappe 18 geöffnet, welche im geschlossenen Zustand den Probenraum 12 bevorzugt lichtdicht verschliesst, damit kein die Untersuchungen beeinflussendes Licht aus der Umgebung in den Probenraum 12 gelangen kann.

Neben dem Aufnehmen von zumindest einer Mikroplatte 2 dient diese Aufnahmeeinrichtung 4 zum Positionieren der Mikroplatte(n) 2 mit den biologischen Zellen oder Zellkulturen enthaltenden Wells 3 gegenüber Aktionsquellen 5',5",5"' und gegenüber Messeinrichtungen 6,7,8 des Mikroplatten-Readers 1.

Der abgebildete Mikroplatten-Reader 1 umfasst des Weiteren zumindest eine Aktionsquelle 5',5",5"' zum Herbeiführen einer Wechselwirkung zwischen zumindest einer dieser Aktionsquellen 5',5",5"' und biologischen Zellen oder Zellkulturen in bestimmten Wells 3 der Mikroplatte(n) 2 und zum Hervorrufen oder Erzeugen eines messbaren Signals. Diese Aktionsquellen sind jedem Fachmann bekannt und sind vorzugsweise ausgewählt aus einer Gruppe, die eine Lichtquelle 5' zum Anregen einer Fluoreszenz in oder an biologischen Zellen in Wells 3 dieser Mikroplatte(n) 2 umfasst. Weitere an sich bekannte Aktionsquellen sind vorzugsweise ausgewählt aus einer Gruppe, die eine Lichtquelle 5' zum Durchstrahlen von biologischen Zellen oder Zellkulturen in Wells (3) dieser Mikroplatte(n) (2) umfasst. Derartige Lichtquellen sind beispielsweise ausgewählt aus einer Gruppe, die Lichtbogenlampen, Blitzlampen, Glühlampen (wie z.B. Halogenlampen), Laser, Laserdioden und Leuchtdioden (LEDs) umfasst. Die entsprechenden Wellenlängen zum Anregen der Fluoreszenz sowie die entsprechenden Fluorophore und deren Emissionscharakteristika sind dem Fachmann ebenfalls bekannt und werden je nach Anwendung ausgewählt. Auch das nicht-invasive Durchstrahlen von Zellen oder Zellkulturen zum Erfassen der Absorbance sowie die dazu zu verwendenden Lichtquellen sind jedem Fachmann geläufig.

Zusätzliche an sich bekannte, jedoch nicht unter die Erfindung fallende Aktionsquellen sind vorzugsweise ausgewählt aus einer Gruppe, die eine Stromquelle 5" und damit auch ein Impedanz-Messeinrichtung zum Messen einer Impedanz oder Impedanz-Änderung in Abhängigkeit einer Anlagerung oder Umlagerung von biologischen Zellen oder Zellkulturen in Wells 3 dieser Mikroplatte(n) 2 umfasst. Derartige Aktionsquellen, welche vorzugsweise eine Wechselstromquelle ist, deren elektrischer Fluss entsprechend einer Sinusfunktion im Bereich zwischen 20 und 100'000 Hz verändert werden kann, wobei die Wechselstromquelle einen Ausgangswiderstand umfasst, sind dem Fachmann beispielsweise aus der Arbeit von Giaever & Keese ("Micromotion of mammalian cells measured electrically", PNAS 1991 Vol. 88: 7896-7900) bekannt. Verwendet werden bevorzugt Mikroplatten 2 mit bestimmten Wells 3, deren Wellboden 16 zumindest teilweise mit Elektroden 17',17" belegt sind, wobei die Elektroden 17',17" bevorzugt mit einer entsprechenden Stromquelle 5" des Mikroplatten-Readers 1 kontaktierbar ausgebildet sind. Für diese Kontaktierung können elektrische Kontakte 23',23" auf den Innenseiten der Wellwände aufgebracht und die Elektroden 17',17" auf diese Weise mit Kontaktfühlern 27 der Wechselstromquelle also mit der Impedanz-Messeinrichtung 5" in Kontakt bringbar ausgebildet sein.
Ebenfalls an sich bekannte Aktionsquellen sind vorzugsweise ausgewählt aus einer Gruppe, die eine Flüssigkeitsquelle 5'" zum Auslösen einer Lumineszenz in oder an biologischen Zellen oder Zellkulturen in Wells 3 dieser Mikroplatte(n) 2 umfasst.

Vorzugsweise ist die Flüssigkeitsquelle 5'" ausgewählt aus einer Gruppe, die Einzelinjektoren und Mehrfachinjektoren zum beispielsweisen Injizieren von Aktivator-Reagenzien zu den Zellen oder Zellkulturen in bestimmten Wells 3 der Mikroplatten 2 umfasst. Derartige Flüssigkeitsquellen 5'" oder Injektoren, die dabei verwendeten, Lumineszenz auslösenden Reagenzien und die Wellenlängen der beispielsweise von Zellen oder deren Stoffwechselprodukten erzeugten Lumineszenz sind dem Fachmann ebenfalls bekannt. Zudem können mit derartigen Injektoren zu den biologischen Zellen oder Zellkulturen in den Wells 3 von Mikroplatten 2 beispielweise auch Stopp-Agenzien oder Nährstoffe zugegeben werden um das Zellwachstum bzw. die Vermehrung der Zellen zu beeinflussen.
Der erfindungsgemässe Mikroplatten-Reader 1 umfasst zudem zumindest eine Messeinrichtung 6,7,8 zum Erfassen zumindest eines integralen Signals, das durch die Aktionsquelle(n) 5', 5'" in oder an biologischen Zellen oder Zellkulturen in den bestimmten Wells 3 der Mikroplatte(n) 2 hervorgerufen oder erzeugt wurde. Derartige Messeinrichtungen sind bevorzugt ausgewählt ist aus einer Gruppe, die Photomultiplier, Photodioden, Photodioden-Arrays, Avalanche-Dioden und phasensensitive Lock-in-Verstärker umfasst. Die Messeinrichtungen 6,8 und Lichtquellen 5' bzw. deren optischer Eingang und/oder Ausgang sind vorzugsweise über Lichtleiter 28, wie optische Fasern oder optische Faserbündel, gekoppelt. Der in der Fig. 1 abgebildete Probenraum 12 ist lichtdicht ausgebildet.
Der erfindungsgemässe Mikroplatten-Reader 1 ist insbesondere dadurch gekennzeichnet, dass er auch eine Beleuchtungsquelle 9 zum Beleuchten und eine Bildgebende Kamera 10 zum Abbilden der Zellen oder Zellkulturen in diesen bestimmten Wells 3 der Mikroplatte(n) 2 umfasst. Speziell bevorzugt ist, dass der Mikroplatten-Reader 1 eine mikroskopische Optik 19 und eine damit optisch gekoppelte Bildgebende Kamera 10 zum Erstellen von mikroskopischen Aufnahmen von biologischen Zellen oder Zellkulturen in den bestimmten Wells 3 der Mikroplatte(n) 2 umfasst. Speziell bevorzugt ist ein erfindungsgemässer Mikroplatten-Reader 1, in den ein Modul mit Bildgebenden Eigenschaften integriert ist, so dass biologische Zellen und Zellkulturen, die sich beispielsweise auf dem Boden der Mikroplattenwells befinden, mit mikroskopischer Auflösung visualisiert werden können. Dieses Bildgebende Modul umfasst eine Beleuchtungseinheit das die Anwendung unterschiedlicher Beleuchtungs- und Abbildungsmodi erlaubt, wie Hellfeldbeleuchtung, Dunkelfeldbeleuchtung und Schrägbeleuchtung. Zusätzliche Modi, die das Aufnehmen von wie Phasenkontrast- oder Fluoreszenz-Bildern erlauben sind ebenfalls denkbar. Die Aufnahme der Bilder erfolgt vorzugsweise über eine mikroskopische Optik 19 mit einer Digitalkamera, die mit einem CMOS- oder CCD-Sensor ausgerüstet ist.
Das bevorzugte Abbildungssystem umfasst zudem eine Autofokuseinrichtung, wobei beim Verwenden von mittleren Aperturen zum Erzielen einer mittleren Auflösung ein Bewegen der Aufnahmeeinrichtung 4 in Richtung einer optischen Achse 20 der Bildgebende Kamera 10 verwendet werden kann. Erfindungsgemäß wird die Konfluenz von Zellkulturen in den Wells 3 von Mikroplatten 2 abgebildet, wobei unter dem Begriff Konfluenz von Zellkulturen auf den Innenoberflächen eines Mikroplattenwells 3 das Verhältnis der von einer Zellkultur bedeckten Fläche zur noch freien Fläche der mit Zellen belegbaren Innenoberfläche dieses Mikroplattenwells 3 wiedergibt.
Der erfindungsgemässe Mikroplatten-Reader 1 ist zudem dadurch gekennzeichnet, dass er einen internen bzw. integrierten Prozessor 11' (vgl. Fig. 2 und 3) umfasst. Ein derartiger Prozessor 11' kann somit ein in die elektronische Steuerung des Mikroplatten-Readers 1 integrierter Mikroprozessor sein. Wichtig ist, dass der Prozessor 11' zum Vergleichen jedes der erfassten integralen Signale mit dem Bild der biologischen Zellen in den entsprechenden Wells 3 der Mikroplatte(n) 2 und zum in eine Relation setzen dieser integralen Signale mit der abgebildeten Konfluenz dieser biologischen Zellen oder Zellkulturen ausgebildet ist. Vorzugsweise wird ein solcher Prozessor 11' durch das Aktivieren einer geeigneten Bildverarbeitungssoftware und durch das Aktivieren einer geeigneten Signalverarbeitungssoftware zum Ausführen dieser Arbeit befähigt.

Das mit der Hilfe dieses Mikroplatten-Readers ausgeführte Verfahren zum Untersuchen von biologischen Zellen umfasst die in Anspruch 1 definierten Schritte, umfassend: Das Aufnehmen von zumindest einer Mikroplatte 2 mit biologischen Zellen oder Zellkulturen enthaltenden Wells 3 mit einer Aufnahmeeinrichtung 4 des Mikroplatten-Readers 1. Üblicherweise wird nur ein Mikroplatte mit den Dimensionen einer Standardmikroplatte, wie diese durch die Society for Biomolecular Sciences (SBS) und dem American National Standards Institute (ANSI) festgelegt worden sind, in die Aufnahmeeinrichtung 4 eingelegt. Abweichend von der Darstellung in den beiliegenden Figuren können aber auch zwei oder mehr Mikroplatten von derselben oder unterschiedlichen Aufnahmeeinrichtungen 4 des Mikroplatten-Readers 1 aufgenommen und transportiert bzw. positioniert werden. Dabei ist diese Aufnahmeeinrichtung 4 bevorzugt als Rahmen ausgebildet, der Anschläge umfasst gegen welche eine aufgenommene Mikroplatte 2 mittels gefederten Andruckmitteln in einer definierten Position gehalten werden. Besonders bevorzugt sind dabei Andruckmittel, die von Hand oder durch einen Robotarm eines Mikroplatten-Handling-Roboters in eine Offenposition zum Einlegen oder Herausnehmen einer Mikroplatte gebracht werden können. Vorzugsweise ist die Aufnahmeeinrichtung 4 in einer X-Richtung, also vorzugsweise horizontal und rechtwinklig zur Klappe 18 motorisch bewegbar. Das Positionieren der Aufnahmeeinrichtung 4 mit den biologischen Zellen oder Zellkulturen enthaltenden Wells 3 der Mikroplatte(n) 2 gegenüber Aktionsquellen 5',5'" des Mikroplatten-Readers 1. Dank einer vorzugsweise definierten Position der Mikroplatte(n) 2 auf der Aufnahmeeinrichtung 4 kann jedes einzelne Well 3 gezielt und individuell gegenüber der Wirkungsachse 21 einer der Aktionsquellen 5',5'" ausgerichtet werden. Vorzugsweise ist die Aufnahmeeinrichtung 4 in einer X-Richtung, also vorzugsweise horizontal und rechtwinklig zur Klappe 18 (vgl. Pfeil in Fig. 1, stellvertretend für alle Fig. 1-3) sowie in einer Y-Richtung, also vorzugsweise horizontal und parallel zur Klappe 18 motorisch bewegbar, so dass jedes Well 3 einer Mikroplatte in einem durch diese beiden Richtungen vorgegeben Feld an praktisch jedem Ort positioniert werden kann. Diese Bewegungen in der X- und Y-Richtung werden bevorzugt von einer zentralen Steuereinheit 22 des Mikroplatten-Readers 1 überwacht und gesteuert.
Das Herbeiführen einer Wechselwirkung zwischen zumindest einer dieser Aktionsquellen 5',5'" und biologischen Zellen oder Zellkulturen in bestimmten Wells 3 der Mikroplatte(n) 2 zum Hervorrufen oder Erzeugen eines messbaren Signals. Entsprechend einem vorzugsweise vorher festgelegten Protokoll aktiviert die zentrale Steuereinheit 22 des Mikroplatten-Readers 1 die entsprechende Aktionsquelle 5',5'" wenn ein bestimmtes Well 3 der Mikroplatte(n) 2 so positioniert ist, dass die Wirkungsachse 21 der entsprechenden Aktionsquelle 5',5'" das bestimmte Well 3 der Mikroplatte(n) 2 trifft. Vorzugsweise wird bei der Durchführung des erfindungsgemässen Verfahrens die zumindest eine Aktionsquelle 5',5'" ausgewählt aus einer Gruppe, die umfasst:
- eine Lichtquelle 5' zum Anregen einer Fluoreszenz in oder an biologischen Zellen oder Zellkulturen in Wells 3 dieser Mikroplatte(n) 2;
- eine Lichtquelle 5' zum Durchstrahlen von biologischen Zellen oder Zellkulturen in Wells 3 dieser Mikroplatte(n) 2; und
- eine Flüssigkeitsquelle 5'" zum Auslösen einer Lumineszenz in oder an biologischen Zellen oder Zellkulturen in Wells 3 dieser Mikroplatte(n) 2.
Dabei ist die Lichtquelle 5' bevorzugt ausgewählt aus einer Gruppe, die Lichtbogenlampen, Blitzlampen, Glühlampen (wie z.B. Halogenlampen), Laser, Laserdioden und Leuchtdioden (LEDs) umfasst. Die Flüssigkeitsquelle 5'" ist vorzugsweise ausgewählt aus einer Gruppe, die Einzelinjektoren und Mehrfachinjektoren sowie die damit verbundenen Reagenzreservoirs und Förderpumpen umfasst. Während dem Herbeiführen einer Wechselwirkung oder unmittelbar danach erfolgt das Positionieren der Aufnahmeeinrichtung 4 mit den biologischen Zellen oder Zellkulturen enthaltenden Wells 3 der Mikroplatte(n) 2 gegenüber Messeinrichtungen 6,7,8 des Mikroplatten-Readers 1 und das Erfassen zumindest eines integralen Signals, das durch die Aktionsquelle(n) 5',5'" in oder an biologischen Zellen oder Zellkulturen in den bestimmten Wells 3 der Mikroplatte(n) 2 hervorgerufen oder erzeugt wurde, mit zumindest einer der Messeinrichtungen 6,7,8:
- Im Fall von Fluoreszenzmessungen in bestimmten Wells 3 von zumindest einer in den Mikroplatten-Reader 1 eingesetzten Mikroplatte 2 wird bevorzugt, dass die entsprechende Messeinrichtung 8 zum Detektieren der Fluoreszenz und zum Erfassen eines integralen Signals ausgewählt ist aus einer Gruppe, die Photomultiplier, Photodioden, Photodioden-Arrays und Avalanche-Dioden umfasst. Bevorzugt weist die Messeinrichtung 8 eine mit der Wirkungsachse 21 der Lichtquelle 5' zum Anregen der Fluoreszenz gemeinsame optische Achse 21' auf; wobei die Lichtquelle 5' und die Messeinrichtung 8 bzw. deren optischer Eingang und Ausgang (vgl. Fig. 1) vorzugsweise über (Top Excitation / Top Reading) bzw. Lichtquelle 5' und Messeinrichtung 8 unter (Bottom Excitation / Bottom Reading) der eingesetzten Mikroplatte 2 angeordnet sind. Geringe Abweichungen der Wirkungsachse 21 von der optischen Achse 21' sind tolerierbar.
- Im Fall von Lumineszenzmessungen in bestimmten Wells 3 von zumindest einer in den Mikroplatten-Reader 1 eingesetzten Mikroplatte 2 wird bevorzugt, dass die entsprechende Messeinrichtung 7 zum Detektieren der Lumineszenz und zum Erfassen eines integralen Signals ausgewählt ist aus einer Gruppe, die Photomultiplier, Photodioden, Photodioden-Arrays und Avalanche-Dioden umfasst. Bevorzugt weist die Messeinrichtung 7 eine mit der Wirkungsachse 21 der Flüssigkeitsquelle 5'" zum Auslösen der Lumineszenz unterschiedliche optische Achse 21' auf; wobei die Flüssigkeitsquelle 5'" vorzugsweise über und die Messeinrichtung 7 vorzugsweise ebenfalls über der eingesetzten Mikroplatte 2 angeordnet sind.
- Im Fall von Absorbancemessungen in bestimmten Wells 3 von zumindest einer in den Mikroplatten-Reader 1 eingesetzten Mikroplatte 2 wird bevorzugt, dass die entsprechende Messeinrichtung 6 zum Detektieren der Absorbance und zum Erfassen eines integralen Signals ausgewählt ist aus einer Gruppe, die Photomultiplier, Photodioden, Photodioden-Arrays und Avalanche-Dioden umfasst. Bevorzugt weist die Messeinrichtung 6 eine mit der Wirkungsachse 21 der Lichtquelle 5' zum Anregen der Fluoreszenz gemeinsame optische Achse 21'" auf; wobei die Lichtquelle 5' vorzugsweise über und die Messeinrichtung 6 bzw. deren optischer Eingang (vgl. Fig. 1) vorzugsweise unter der eingesetzten Mikroplatte 2 angeordnet sind.

Das erfindungsgemässe Verfahren zum Untersuchen von biologischen Zellen oder Zellkulturen in einem Mikroplatten-Reader 1 umfasst einerseits, dass mit einer Beleuchtungsquelle 9 des Mikroplatten-Readers 1 die biologischen Zellen oder Zellkulturen in den bestimmten Wells 3 der Mikroplatte(n) 2 beleuchtet werden, wobei in diesen bestimmten Wells 3 zuvor oder danach ein integrales Signal mit einer Messeinrichtung 6,7,8 erfasst wird. Das erfindungsgemässe Verfahren zum Untersuchen von biologischen Zellen oder Zellkulturen in einem Mikroplatten-Reader 1 umfasst andererseits, dass mit einer Bildgebenden Kamera 10 des Mikroplatten-Readers 1 Bilder der biologischen Zellen oder Zellkulturen in diesen bestimmten Wells 3 der Mikroplatte(n) (2) erstellt werden. Insbesondere ist das erfindungsgemässe Verfahren zum Untersuchen von biologischen Zellen oder Zellkulturen in einem Mikroplatten-Reader 1 jedoch dadurch gekennzeichnet, dass mit einem Prozessor 11' jedes der erfassten integralen Signale mit dem vorher oder nachher aufgenommenen Bild der biologischen Zellen in den entsprechenden Wells 3 der Mikroplatte(n) 2 verglichen und mit der abgebildeten Konfluenz dieser biologischen Zellen oder Zellkulturen in Relation gesetzt wird.
Die Figur 2 zeigt einen Vertikalschnitt durch einen Mikroplatten-Reader 1 gemäss einer zweiten Ausführungsform beim Erfassen von integralen Signalen in ausgewählten Wells 3 von Mikroplatten 2 bzw. beim mikroskopischen Abbilden von Mikroplattenwells 3 in einem vorzugsweise als lichtdicht und gasdicht abschliessbare Isolierkammer ausgebildeten Probenraum 12. Während in der ersten Ausführungsform von Fig. 1 nur das Vermeiden von störendem Umgebungslicht im Vordergrund steht, so ist diese zweite Ausführungsform neben dem Vermeiden von störendem Umgebungslicht darauf ausgerichtet, den Zellen oder Zellkulturen in den Wells 3 von Mikroplatten 2 eine speziell geeignete Atmosphäre anzubieten.

Vorzugsweise umfasst der Mikroplatten-Reader 1 ein Kontrollgerät 13, mit welchem in dem als Isolierkammer ausgebildeten Probenraum 12 des Mikroplatten-Readers 1 eine kontrollierte Atmosphäre hergestellt wird, indem die Konzentration der anwesenden Gase, die ausgewählt sind aus eine Gruppe, die Sauerstoff, Stickstoff, Kohlendioxid und Kohlenmonoxid umfasst, in je einem bestimmten Wertebereich geregelt oder gehalten wird. Dieses Kontrollgerät 13 kann in den Mikroplatten-Reader 1 integriert oder diesem beigestellt oder aufgesetzt sein. Im Probenraum 12 sind beispielsweise ein O₂-Sensor zur Messung und Steuerung des Sauerstoffgehalts der Gasatmosphäre um die Zellen oder Zellkulturen enthaltenden Wells 3 von in diesen Mikroplatten-Reader 1 eingesetzten Mikroplatten 2 und ein CO₂-Sensor zur Messung und Steuerung des Kohlendioxidgehalts dieser Gasatmosphäre angeordnet. Vorzugsweise sind Gaseinlässe 24 und Gasauslässe 25 vorgesehen, welche die thermisch isolierten Wände oder Boden des Probenraums 12 durchstechen und mit dem Kontrollgerät 13 verbunden sind oder verbunden werden können. Zum Bewegen sowie Mischen und Verteilen der im Probenraum anwesenden Gase können ein Lüfter (nicht gezeigt) oder andere Misch-Vorrichtungen im Probenraum 12 vorgesehen werden.

In Falle eines separaten Kontrollgeräts 13 ist dieses vorzugsweise direkt an die notwendigen Druckflaschen für die zu verwendenden Gase angeschlossen. Die entsprechenden Ventile und Drosseln für die benötigten Gasanschlüsse sind in dieses separate Kontrollgerät 13 eingebaut (nicht gezeigt). Alternativ können auch die Ventile und Drosseln auf den Gasdruckflaschen verwendet werden, wobei diese Ventile vorzugsweise als elektrisch gesteuerte Magnetventile (des Typs stromlos geschlossen) ausgebildet sind. Nicht gezeigt in der Fig. 2 sind unter anderem die Bedienungselemente, Anzeigeelemente und Stromversorgungen für das separate oder in den Mikroplatten-Reader 1 integrierte Kontrollgerät 13 und den Mikroplatten-Reader 1 selbst.

Die Kontrolleinheit umfasst vorzugsweise einen Rechner 28 mit der entsprechenden Software; dabei kann der Rechner 28 mit einem zentralen Rechner 29 des Mikroplatten-Readers 1 verbunden (vgl. Fig. 1), verbindbar (z.B. in einem separaten Gehäuse 17' untergebracht) oder in diesen zentralen Rechner 29 des Mikroplatten-Readers 1 integriert sein (nicht gezeigt).

Es kann vorgesehen sein, dass zum Verdrängen der Umgebungsluft im Probenraum 12 Stickstoff (N₂) und/oder Kohlendioxyd (CO₂) verwendet wird, wobei entsprechende Druckflaschen bereitgestellt werden. Diese Druckflaschen sind wie üblich mit an sich bekannte Regelventile und Drosseln ausgerüstet, um den benötigten Lieferdruck für diese Gase einzustellen. Alternativ dazu können derartige Prozessgase auch aus anderen Quellen (z.B. aus Hausleitungen) bezogen werden. Neben Stickstoff und Kohlendioxyd können (kombiniert mit entsprechenden Gasdetektoren im Probenraum 12) auch andere Gase zum Erzeugen eines definierten Anteils an der normalerweise im Probenraum vorherrschenden Atmosphäre eingesetzt werden. Bei Beachtung der unter Umständen geltenden Vorsichtsmassnahmen können somit auch andere Gase, wie beispielsweise Edelgase bzw. inerte Gase (z.B. Argon) oder auch reaktive oder giftige Gase (z.B. Sauerstoff, Kohlenmonoxid, Schwefelwasserstoff oder Schwefeldioxid) zum Erzeugen einer bestimmten Zusammensetzung der Gasatmosphäre über den bzw. in der Umgebung der Wells 3 von in diesen Mikroplatten-Reader 1 eingesetzten Mikroplatten 2 über mindestens einen Gaseinlass 24 in den Probenraum 12 des Mikroplatten-Readers 1 eingeleitet werden. Vorzugsweise ist das Kontrollgerät mit genügend Gaszuleitungen und Steuerventilen ausgerüstet, so dass auch komplexere Gaszusammensetzungen mit mehreren GasKomponenten ermöglicht werden.

Stellvertretend für geeignete CO₂-Sensoren soll der CO₂-Sensor SenseAir® CO₂ Engine® ICB, Part No.: 033-9-0001 der Firma SenseAir AB in SE-820 60 Delsbo, Schweden, genannt werden. Stellvertretend für geeignete O₂-Sensoren soll der O₂-Sensor Pewatron FCX-MEP2-F-CH Sauerstoffmodul der Firma Pewatron AG in CH-8052 Zürich, Schweiz, genannt werden.

Vorzugsweise ist der als Isolierkammer ausgebildete Probenraum 12 mit einem Luftkühlsystem ausgerüstet, das über Zu- und Ableitungen mit dem Probenraum 12 verbunden ist. Das Kontrollgerät 13 kann zusätzlich als derartiges Luftkühlsystem ausgebildet sein und Kühlelemente, z.B. in Form von Peltierelementen, umfassen. In besonders bevorzugten Mikroplatten-Readern 1 kann mit einem Kontrollgerät 13 in dem als Isolierkammer ausgebildeten Probenraum 12 eine kontrollierte Atmosphäre hergestellt werden, indem Parameter, die ausgewählt sind aus einer Gruppe, die Temperatur, relative Feuchtigkeit und Totaldruck umfassen, in je einem bestimmten Wertebereich geregelt oder gehalten werden. Bevorzugt wird dabei die relative Feuchtigkeit in einem Endfeuchte-Bereich gehalten oder geregelt, so dass keine Kondensation von Wasserdampf an Oberflächen im Probenraum und auch kein Austrocknen der Zellen oder Zellkulturen befürchtet werden muss.
Im Zusammenhang mit der vorliegenden Erfindung werden Zellkulturen, von solchen abgetrennte oder sonstwie gewonnene biologische Zellansammlungen oder Einzelzellen als Zellen bezeichnet, wobei diese Zellen Mikroorganismen und Pilze sowie tierische und pflanzliche Eukaryotenzellen umfassen.
In Fig. 2 befindet sich gerade ein Well 3 im Bereich der optischen Achse 21" der zweiten Messeinrichtung 7 für Lumineszenzmessungen. Ein weiteres Well 3 befindet sich gerade in Bereich der optischen Achse 20 der Bildgebenden Kamera 10 und der Beleuchtungsquelle 9. Ein anderes Well 3 der gleichen Mikroplatte 2 befindet sich gerade im Bereich der optischen Achse 21"' der ersten Messeinrichtung 6 für Absorbancemessungen. Während in der Fig. 1 zwei Bildgebende Kameras 10 mit den dazu gehörenden mikroskopischen Optiken 19 und Beleuchtungsquellen 9 gezeigt sind, weist die zweite Ausführungsform nur eine entsprechend ausgerüstete Bildgebende Kamera 10 auf.
Die Figur 3 zeigt einen Vertikalschnitt durch einen Mikroplatten-Reader 1 gemäss einer dritten Ausführungsform beim Erfassen von integralen Signalen in ausgewählten Mikroplattenwells 3 in einem vorzugsweise als lichtdicht und gasdicht abschliessbare Isolierkammer ausgebildeten Probenraum 12. Diese dritte Ausführungsform des erfindungsgemässen Mikroplatten-Readers 1 weist ein Gehäuse 14, in das der gesamte Mikroplatten-Reader 1 mit allen Aktionsquellen 5',5'" und Messeinrichtungen 6,7,8 eingebaut ist. Zudem ist in das Gehäuse 14 neben dem als Isolationskammer ausgebildeten Probenraum 12 auch eine zentrale Steuereinheit 22 eingebaut, wie sie auch von den ersten beiden Ausführungsformen umfasst wird. In Fig. 3 befindet sich gerade ein Well 3 im Bereich der Wirkungsachse 21 der Flüssigkeitsquelle 5"', die (wie auch in den Fig. 1 und 2) mit einem Doppel-Injektor ausgerüstet ist. Ein weiteres Well 3 befindet sich gerade in Bereich der optischen Achse 21" der zweiten Messeinrichtung 7 für Lumineszenzmessungen. Eine möglichst nahe örtliche Anordnung zumindest eines Injektors einer Flüssigkeitsquelle 5"' zur optischen Achse 21" der zweiten Messeinrichtung 7 ist bevorzugt, damit die Messung der Lumineszenz mit möglichst kleinen Zeitdifferenzen zwischen Injektion (Auslösen der Lumineszenz) und Detektion der Lumineszenz erfolgen kann. Unweit von der optischen Achse 21" der zweiten Messeinrichtung 7 ist auch die optische Achse 20 der Bildgebenden Kamera 10 mit der mikroskopischen Optik 19 und der Beleuchtungsquelle 9 angeordnet.
Bevorzugt umfasst der Mikroplatten-Reader 1 einen Bildschirm 15 zur Anzeige von Betriebszuständen, Messresultaten, mikroskopischen Bildern und zur Anzeige von Resultaten aus dem Vergleich der erfassten integralen Signale mit den entsprechenden Bildern der biologischen Zellen oder Zellkulturen in den jeweiligen Wells 3 der Mikroplatte(n) (2) und dem Verhältnis zu der Konfluenz dieser biologischen Zellen oder Zellkulturen. Bevorzugt ist der Bildschirm 15 als berührungsempfindlicher Bildschirm oder Touch Screen ausgebildet, so dass er auch als Interface zum Einstellen oder Verändern der Betriebsparameter des Mikroplatten-Readers 1 sowie zum Bearbeiten und Speichern der erzeugten Resultate verwendet werden kann.

Die Figur 4 zeigt eine Aufsicht auf nicht erfindungsgemäße Mikroplattenwells 3 mit auf dem Wellboden 16 aufgebrachten, beispielhaften Elektroden 17',17" und mit auf den Wellwänden aufgebrachten, beispielhaften elektrischen Kontakten 23',23". Diese Elektroden 17',17" und elektrischen Kontakte 23',23" sind vorzugsweise aus Gold oder einer Goldlegierung hergestellt und mittels Sputtern oder einer anderen geeigneten Beschichtungsmethode auf den Wellboden 16 bzw. auf die Innenoberfläche und vorzugsweise auch auf den Wellrand 26 aufgebracht. Insbesondere die bis auf den Wellrand 26 reichenden elektrischen Kontakte 23',23" können relativ einfach mit den Kontaktfühlern 27 der Stromquelle 5" verbunden werden, indem die Mikroplatte 2 mit der Aufnahmeeinrichtung 4 gegen die Stromquelle 5" hin, also vorzugsweise mit einem entsprechenden motorischen Antrieb in einer vertikalen Z-Richtung bewegt wird (vgl. Pfeil in Fig. 1, stellvertretend für alle Fig. 1-3), bis die Kontaktfühler 27 der Stromquelle 5" mit den elektrischen Kontakten 23',23" berührt werden. Vorzugsweise wird also die Aufnahmeeinrichtung 4 mit den biologischen Zellen oder Zellkulturen enthaltenden Mikroplatte(n) 2 beim Positionieren in zumindest einer Bewegungsrichtung bewegt, wobei diese Bewegungsrichtung ausgewählt ist aus eine Gruppe, die eine X-, eine Y- und eine Z-Richtung in einem dreidimensionalen Koordinatensystem umfasst. Zum Herbeiführen einer Wechselwirkung zwischen zumindest einer der Aktionsquellen 5',5",5"' und biologischen Zellen oder Zellkulturen in Wells 3 der Mikroplatte(n) 2 aber auch zum Erfassen eines entsprechenden integralen Signals mit zumindest einer der Messeinrichtungen 6,7,8 oder der Stromquelle 5" kann die Aufnahmeeinrichtung 4 mit den biologischen Zellen oder Zellkulturen enthaltenden Mikroplatte(n) 2 bevorzugt in zumindest einer Bewegungsrichtung bewegt werden, wobei diese Bewegungsrichtung ausgewählt ist aus eine Gruppe, die eine X-, eine Y- und eine Z-Richtung in einem dreidimensionalen Koordinatensystem umfasst.

Die Fig. 4 zeigt eine Auswahl von beispielhaften Elektroden 17',17", deren Material die Adhärenz, Konfluenz oder Morphologie von biologischen Zellen oder Zellkulturen möglichst wenig und vorzugsweise gar nicht beeinflusst. Die Elektroden 17',17" können wie gezeigt z.B. gleich oder unterschiedlich ausgebildet sein und eine Fläche vollständig bedecken oder nur ein Gebiet oder Array abdecken.

Die gleichen Bezugszeichen und Signaturen zeigen auf vergleichbare Merkmale hin, auch wenn diese nicht für alle Figuren und alle Bezugszeichen beschrieben sind. Beliebige Kombinationen der beschriebenen und/oder gezeigten Merkmale gehören zum Umfang der vorliegenden Erfindung.

Als Fluoreszenzmessungen im Zusammenhang mit der vorliegenden Erfindung werden unter anderem Messungen der Fluoreszenz-Intensität; Zeitaufgelöste Fluoreszenzmessungen (Time Resolved Fluorescence); Messung der Fluoreszenz-Polarisation und Messung der Fluoreszenz-Lebenszeit (Fluorescence Lifetime) besonders bevorzugt. Zusätzlich zur Intensitätsmessung der Lumineszenz sind im Zusammenhang mit der vorliegenden Erfindung insbesondere auch Kinetik-Messungen an Zellen und Zellkulturen anhand der Lumineszenz aber auch das Erfassen der Lumineszenzkinetik von Interesse.

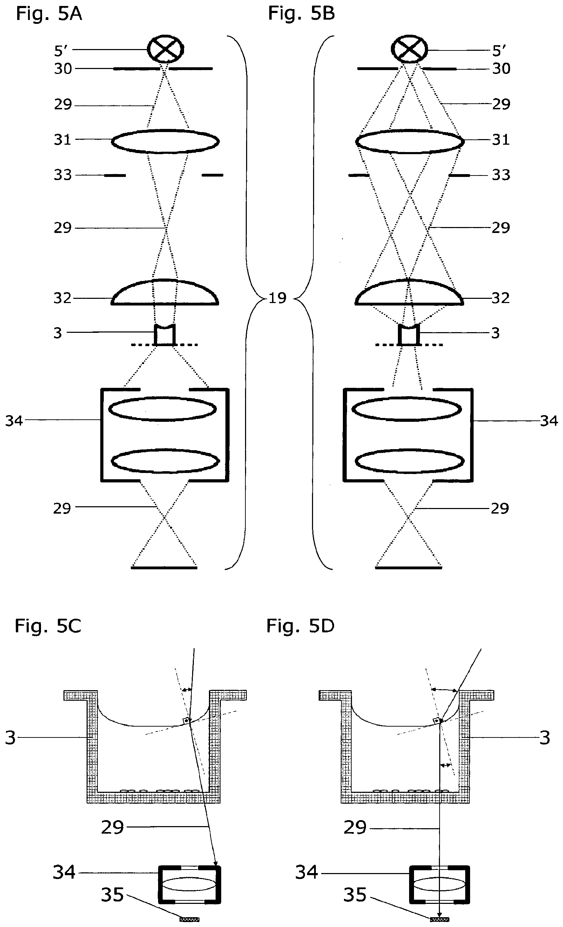

Die Figur 5 zeigt eine schematische Darstellung des Strahlengangs der Durchlicht-Beleuchtung durch ein Well 3 einer Mikroplatte 2. Dabei zeigt Fig. 5A eine konventionelle Durchlicht-Beleuchtung mit eingezeichnetem Verlauf eines transmittierten Lichtstrahlbündels 29. Diese klassische Beleuchtungsart für die Hellfeldmikroskopie wird Köhler'sche Beleuchtung genannt.

Als Lichtquelle 5' dient eine LED. Deren Licht 29 wird von einer Kollektorlinse 31 eingesammelt und in die Brennebene der Kondensorlinse 32 abgebildet. Die Kondensorlinse 32 fokussiert das Licht 29 in die Probenebene und beleuchtet mit hoher Intensität den kleinen Probenbereich im Well 3, welcher vom Objektiv 34 stark vergrößernd auf den CCD- oder CMOS-Chip 35 der Kamera 10 abgebildet wird. Die nach der Kollektorlinse 31 platzierte Leuchtfeldblende 33 begrenzt den beleuchteten Bereich in der Probenebene im Well 3 und hat die Funktion, die Belastung der Probe durch Licht und Wärmestrahlung zu minimieren, sowie Streulicht zu minimieren. Die nach der Lichtquelle 5' platzierte Aperturblende 30 definiert die numerische Apertur des Beleuchtungslichtkegels 29 zwischen Kondensorlinse 32 und Probenebene im Well 3. Ist die Aperturblende 30 klein, so weist der Beleuchtungslichtkegel 29 einen kleinen Öffnungswinkel auf. Ist die Aperturblende 30 hingegen gross, so weist der Beleuchtungslichtkegel einen grossen Öffnungswinkel auf. Normalerweise wird die Aperturblende 30 in der Brennebene der Kondensorlinse 32 platziert. Im gezeigten Aufbau liegt sie aus Platzgründen in der Lichtquellen-Ebene, was von der optischen Abbildung her äquivalent ist. Die gezeigte Platzierung der Aperturblende 30 erlaubt die Verwendung eines motorisch betriebenen Blendenrads (nicht gezeigt), das verschieden grosse und verschieden geformte Aperturblenden 30 enthält.

Im Gegensatz zu einem normalen Mikroskopiepräparat, welches sich dünn und zweidimensional zwischen zwei Glasplättchen (Mikroskopie-Objektträger und Deckglas) befindet, muss beim Mikroskopieren in Wells 3 von Mikroplatten 2 das beleuchtende Licht 29 durch das gesamte Flüssigkeitsvolumen hindurchtreten, bevor es die am Boden des Wells 3 befindliche Probenebene erreicht. Die Flüssigkeitsoberfläche ist durch Adhäsionseffekte deutlich gekrümmt, d.h. ein Meniskus ist ausgebildet, der wie eine Zerstreuungslinse wirkt.

Die Fig. 5C zeigt eine konventionelle Durchlicht-Beleuchtung mit eingezeichnetem Verlauf eines transmittierten Lichtstrahls 29. Bei Beleuchtung mit kleiner Aperturblende 30 (vgl. Fig. 5A), werden die Randstrahlen, welche normalerweise den äusseren Bereich des vom Objektiv 34 abgebildeten Probenbereiches beleuchten, durch die Schrägstellung des Meniskus nach aussen abgelenkt und treffen nicht mehr auf die Aussenbereiche des abgebildeten Bodens des Wells 3 oder werden wie gezeigt vom Kameraobjektiv 34 und vom Bildgebenden Chip 35 nicht erfasst. Im resultierenden Bild ist deshalb ein starker Intensitätsabfall nach aussen die Folge. Dieser lässt sich rechnerisch in der nachfolgenden Bildverarbeitung nur teilweise kompensieren. Der resultierende Kontrastverlust führt dazu, dass die Zellzählung bzw. Konfluenzbestimmung in den Aussenbereichen des Wells weniger zuverlässig ist.

Die Fig. 5B zeigt eine erfindungsgemässe Durchlicht-Beleuchtung mit eingezeichnetem Verlauf eines transmittierten Lichtstrahlbündels 29. Der Aufbau für diese Beleuchtung mit hoher numerischer Apertur ist bis auf die Grösse der Aperturblende 30 identisch mit demjenigen für die normale Beleuchtung (vgl. Fig. 5A). Die Aperturblende 30 wird hier bewusst vergrössert, um den Öffnungswinkel des Beleuchtungslichtkegels 29 zu vergrössern.

Die Fig. 5D zeigt eine erfindungsgemässe Durchlicht-Beleuchtung mit eingezeichnetem Verlauf eines transmittierten Lichtstrahls 29. Die Randstrahlen des Beleuchtungslichtkegels 29 treffen unter einem grösseren Winkel auf die gekrümmte Flüssigkeitsoberfläche, werden daher weniger stark nach aussen abgelenkt und beleuchten so auch die Randbereiche des Bodens des Wells 3 derart, dass das Licht ins Kameraobjektiv 34 eintreten kann und vom Bildgebenden Chip 35 erfasst wird. Dies hat zur Folge dass der Intensitätsgradient deutlich schwächer ausfällt. Der Kontrast ist auch in den Aussenbereichen des Wells besser und eine Zellzählung bzw. Konfluenzbestimmung wird auch in diesen Bereichen zuverlässig möglich.

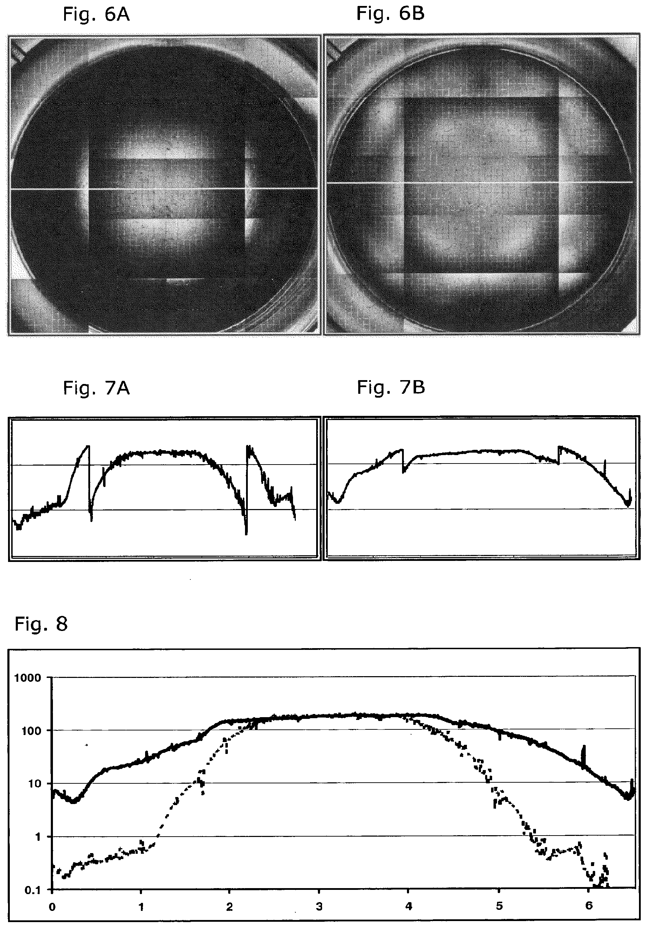

Die Figur 6 zeigt Abbildungen eines Wells 3 in Durchlicht-Beleuchtung. Hier werden als Mosaik zusammengesetzte Aufnahmen eines Wells 3 mit einer Zellkultur gezeigt. Die Helligkeit der Bildaufnahme hängt unter anderem von der Belichtungszeit ab, diese wurde von der Aufnahmesoftware für jedes Einzelbild kontrastoptimiert. Die globale Helligkeitsverteilung über den Boden des Wells 3 führt zu den Helligkeitssprüngen an den Kontaktstellen der Einzelbilder.

Die Figur 6A das Resultat einer konventionellen Beleuchtung. Die Einzelbilder wurden mit kleiner Aperturblende 30 aufgenommen (vgl. Fig. 5A und 5C), der Helligkeitsabfall zum Rand hin ist stark ausgeprägt.

Die Fig. 6B zeigt als Resultat einer erfindungsgemässen Beleuchtung das gleiche Well 3 bei Beleuchtung mit vergrösserter Aperturblende 30 (vgl. Fig. 5B und 5D). Man erkennt deutlich, dass bis zum Wellrand hin auswertbare Helligkeitswerte vorliegen.

Die Figur 7 zeigt die Intensitätsverteilungen in den Abbildungen von Fig. 6 über einen ganzen Durchmesser der Wells 3 (vgl. weisse Linie). Gezeigt ist ein horizontaler Intensitäts-Querschnitt durch die beiden Mosaiken. Die Sprünge resultieren durch die Unterschiede in der Belichtungszeit der Einzelbilder.

Die Fig. 7A zeigt die Intensitätsverteilung bei konventioneller Beleuchtung mit kleiner Aperturblende 30 (vgl. Fig. 5A und 5C). Man erkennt, dass bei normaler Beleuchtung in den Aussenbereichen nur sehr niedrige Intensitätswerte erreicht werden, die im Bereich des Kamerarauschens liegen.

Die Fig. 7B zeigt die Intensitätsverteilung bei erfindungsgemässer Beleuchtung mit hoher numerischer Apertur 30 (vgl. Fig. 5B und 5D). Es ist offensichtlich, dass die Gradienten viel schwächer ausgeprägt sind. Die Intensitäts-Sprünge von einem Einzelbild zum anderen sind viel kleiner, da die durch die Belichtungszeit kompensierten globalen Helligkeitsunterschiede weniger stark sind.

Die Figur 8 zeigt den Vergleich der beiden Abbildungen von Fig. 7A und 7B. Für diesen direkten Vergleich der Resultate der konventionellen und der erfindungsgemässen Abbildung wurden die Unterschiede in den Belichtungszeiten normiert. In der Grafik sind auf der Abszisse Positionen auf dem Wellboden in Abständen von 1 mm angegeben und auf der Ordinate Intensitätswerte (Grauwerte) in logarithmischer Skala angegeben. Die gestrichelte Linie entspricht der konventionellen Beleuchtung mit kleiner Aperturblende und die ausgezogene Linie entspricht der erfindungsgemässen Beleuchtung mit grosser Aperturblende. Der Intensitätsabfall zum Wellrand liegt bei der erfindungsgemässen Beleuchtung mit hoher numerischer Apertur bei etwa 20-fach. Dagegen beträgt der Randabfall bei normaler, herkömmlicher Beleuchtung etwa das 500-fache. Dies führt bei Beleuchtung mit hoher numerischer Apertur zu auswertbaren Signalen im Randbereich, was bei normaler Beleuchtung wie gezeigt nicht möglich ist. Als kleine numerische Apertur wird eine Aperturblende 30 mit einer Öffnung in einem Bereich von 1-3 mm und als hohe numerische Apertur wird eine Aperturblende 30 mit einer Öffnung in einem Bereich von 5-9 mm betrachtet. Speziell bevorzugt beträgt der Öffnungsdurchmesser der Aperturblende 30 bei einer hohen numerischen Apertur 9 mm.

**Bezugszeichenliste**

| | | | |
|---|---|---|---|
| 1 | Mikroplatten-Reader | 17',17" | Elektroden |
| 2 | Mikroplatte | 18 | Klappe |
| 3 | Well | 19 | mikroskopische Optik |
| 4 | Aufnahmeeinrichtung | 20 | optische Achse von 10 |
| 5',5",5"' | Aktionsquellen | 21 | Wirkungsachse |
| 5' | Lichtquelle | 21' | optische Achse von 8 |
| 5" | Stromquelle; Impedanz-Messeinrichtung | 21" | optische Achse von 7 |
| | | 21'" | optische Achse von 6 |
| 5'" | Flüssigkeitsquelle | 22 | zentrale Steuereinheit |
| 6 | erste Messeinrichtung für Absorbancemessungen | 23',23" | elektrische Kontakte |
| | | 24 | Gaseinlässe |
| 7 | zweite Messeinrichtung für Lumineszenzmessungen | 25 | Gasauslässe |
| | | 26 | Wellrand |
| 8 | dritte Messeinrichtung für Fluoreszenzmessungen | 27 | Kontaktfühler |
| | | 28 | Lichtleiter |
| 9 | Beleuchtungsquelle | 29 | Licht, Lichtstrahl, Lichtstrahlbündel |
| 10 | Bildgebende Kamera | | |
| 11' | interner Prozessor | 30 | Aperturblende |
| 11" | externer Prozessor | 31 | Kollektorlinse |
| 12 | Probenraum | 32 | Kondensorlinse |
| 13 | Kontrollgerät | 33 | Leuchtfeldblende |
| 14 | Gehäuse | 34 | Kameraobjektiv |
| 15 | Bildschirm | 35 | CCD, CMOS Chip |
| 16 | Wellboden | | |

## Patentansprüche

1. Verfahren zum Untersuchen von biologischen Zellen oder Zellkulturen in einem Mikroplatten-Reader (1), wobei das Verfahren umfasst:
a) Bereitstellen eines Mikroplatten-Reader (1), welcher umfasst:
- zumindest eine Messeinrichtung (6,7,8) mit einer optischen Achse (21', 21", 21"');
- eine Beleuchtungsquelle (9) und eine bildgebende Kamera (10) mit einer eigenen optischen Achse (20), welche zu der optischen Achse (21', 21", 21"') der Messeinrichtung (6,7,8) parallel verläuft,
- eine Aufnahmeeinrichtung (4) zum Aufnehmen von zumindest einer Mikroplatte (2) und zum Positionieren einer aufgenommenen Mikroplatte (2) gegenüber der optischen Achse (21', 21", 21'") der Messeinrichtung (6,7,8) oder gegenüber der optischen Achse (20) der Beleuchtungsquelle (9) und der bildgebenden Kamera (10) des Mikroplatten-Readers (1); und
- einen Prozessors (11');
b) Positionieren eines Wells (3) einer aufgenommenen Mikroplatte (2) gegenüber der optischen Achse (21', 21", 21'") der Messeinrichtung (6,7,8) des Mikroplatten-Readers (1) mit der Aufnahmeeinrichtung (4), und Erfassen eines integralen Signals in dem einen Well (3) der Mikroplatte (2) mit zumindest einer der Messeinrichtungen (6,7,8), wobei das integrale Signal ausgewählt ist aus einer Gruppe, welche umfasst: eine Fluoreszenz, eine Lumineszenz und eine Absorbance;
**dadurch gekennzeichnet, dass** das Verfahren die folgenden, weiteren Schritte umfasst:
c) Positionieren des einen Wells (3) der Mikroplatte (2) gegenüber der optischen Achse (20) der Beleuchtungsquelle (9) und der bildgebenden Kamera (10) mit der Aufnahmeeinrichtung (4), Durchstrahlen und Erstellen eines Transmissions-Bilds eines Bodens dieses Wells (3) mit der Beleuchtungsquelle (9) und der bildgebenden Kamera (10);
d) Bestimmen einer Konfluenz von biologischen Zellen oder Zellkulturen auf dem in Schritt c) erstellten Transmissions-Bild des Bodens des einen Wells (3),
e) Vergleichen des erfassten integralen Signals in dem einen Well (3) aus Schritt b) mit der bestimmten Konfluenz der biologischen Zellen oder Zellkulturen aus Schritt d) mittels des Prozessors (11'); und
f) Normieren des integralen Signals des einen Wells (3) aus Schritt b) mit der in Schritt d) bestimmten Konfluenz der biologischen Zellen oder Zellkulturen in diesem Well (3).

2. Verfahren nach Anspruch 1, **dadurch gekennzeichnet, dass** die Aufnahmeeinrichtung (4) mit den biologischen Zellen oder Zellkulturen enthaltenden Wells (3) der Mikroplatte(n) (2) gegenüber Aktionsquellen (5',5"') des Mikroplatten-Readers (1) positioniert und mit einer der Aktionsquellen eine Wechselwirkung mit den biologischen Zellen oder Zellkulturen in bestimmten Wells (3) der Mikroplatte(n) (2) zum Hervorrufen oder Erzeugen des integralen Signals erzeugt wird.

3. Verfahren nach Anspruch 2, **dadurch gekennzeichnet, dass** die zumindest eine Aktionsquelle (5',5"') ausgewählt ist aus einer Gruppe, die umfasst:
- eine Lichtquelle (5') zum Anregen einer Fluoreszenz in oder an biologischen Zellen oder Zellkulturen in Wells (3) dieser Mikroplatte(n) (2);
- eine Lichtquelle (5') zum Durchstrahlen von biologischen Zellen oder Zellkulturen in Wells (3) dieser Mikroplatte(n) (2); und
- eine Flüssigkeitsquelle (5'") zum Auslösen einer Lumineszenz in oder an biologischen Zellen oder Zellkulturen in Wells (3) dieser Mikroplatte(n) (2).

4. Verfahren nach Anspruch 3, **dadurch gekennzeichnet, dass** die Lichtquelle (5') ausgewählt ist aus einer Gruppe, die Lichtbogenlampen, Blitzlampen, Glühlampen, Laser, Laserdioden und Leuchtdioden (LEDs) umfasst.

5. Verfahren nach Anspruch 3, **dadurch gekennzeichnet, dass** die Flüssigkeitsquelle (5"') ausgewählt ist aus einer Gruppe, die Einzelinjektoren und Mehrfachinjektoren umfasst.

6. Verfahren nach Anspruch 1, **dadurch gekennzeichnet, dass** die Messeinrichtungen (6,7,8) zum Erfassen eines integralen Signals ausgewählt sind aus einer Gruppe, die Photomultiplier, Photodioden, Photodioden-Arrays, Avalanche-Dioden und phasensensitive Lock-in-Verstärker umfasst.

7. Verfahren nach Anspruch 1, **dadurch gekennzeichnet, dass** die Aufnahmeeinrichtung (4) mit den biologischen Zellen oder Zellkulturen enthaltenden Mikroplatte (2) beim Positionieren in zumindest einer Bewegungsrichtung bewegt wird, wobei diese Bewegungsrichtung ausgewählt ist aus eine Gruppe, die eine X-, eine Y- und eine Z-Richtung in einem dreidimensionalen Koordinatensystem umfasst.

8. Verfahren nach Anspruch 1, **dadurch gekennzeichnet, dass** die mit der Aufnahmeeinrichtung (4) aufgenommenen biologische Zellen oder Zellkulturen enthaltenden Wells (3) der Mikroplatte (2) in einem lichtdichten Probenraum (12) des Mikroplatten-Readers (1) untersucht werden.

9. Verfahren nach Anspruch 1, **dadurch gekennzeichnet, dass** die mit der Aufnahmeeinrichtung (4) aufgenommenen biologische Zellen oder Zellkulturen enthaltenden Wells (3) der Mikroplatte (2) in einem als Isolierkammer ausgebildeten Probenraum (12) des Mikroplatten-Readers (1) einer kontrollierten Atmosphäre ausgesetzt werden.

10. Verfahren nach Anspruch 8 oder 9, **dadurch gekennzeichnet, dass** die aufgenommene Mikroplatte (2) mit den biologische Zellen oder Zellkulturen enthaltenden Wells (3) zumindest während dem Durchführen der Verfahrensschritte b) und c) in dem Probenraum (12) des Mikroplatten-Readers (1) von der Aufnahmeeinrichtung (4) gehalten werden.

11. Verfahren nach einem der vorhergehenden Ansprüche, **dadurch gekennzeichnet, dass** zum Abbilden des Bodens des Wells (3) eine mikroskopische Optik (19) verwendet wird, die eine Aperturblende (30) mit hoher numerischer Apertur umfasst.

12. Verfahren nach Anspruch 1, **dadurch gekennzeichnet, dass** alle Verfahrensschritte a) bis f) in einem einzigen Gerät automatisiert durchgeführt werden.

13. Mikroplatten-Reader (1), der umfasst:
a) zumindest eine Messeinrichtung (6,7,8) zum Erfassen eines integralen Signals, das in oder an biologischen Zellen oder Zellkulturen in einem Well (3) einer Mikroplatte(n) (2) existiert, hervorgerufen oder erzeugt wurde, wobei die Messeinrichtung (6,7,8) eine optische Achse (21', 21", 21"') umfasst, und wobei das integrale Signal ausgewählt ist aus einer Gruppe, welche umfasst: eine Fluoreszenz, eine Lumineszenz und eine Absorbance;
b) eine Beleuchtungsquelle (9) und eine bildgebende Kamera (10) zum Durchstrahlen eines Wells (3) einer Mikroplatte (2) und zum Erstellen eines Transmissions-Bilds eines Bodens dieses Wells (3), wobei die Beleuchtungsquelle (9) und die bildgebende Kamera (10) eine optische Achse (20) umfasst, welche zu der optischen Achse (21', 21", 21'") der Messeinrichtung (6,7,8) parallel verläuft, und
c) eine Aufnahmeeinrichtung (4) zum Aufnehmen von zumindest einer Mikroplatte (2) mit Wells (3) sowie zum Positionieren der Mikroplatte (2) gegenüber der optischen Achse (21', 21", 21"') der Messeinrichtung (6,7,8) oder gegenüber der optischen Achse (20) der Beleuchtungsquelle (9) und der bildgebenden Kamera (10);
**dadurch gekennzeichnet, dass** der Mikroplatten-Reader (1) einen internen Prozessor (11') umfasst, wobei der Prozessor (11') ausgebildet ist:
- zum Vergleichen eines erfassten integralen Signals eines Wells (3) einer von der Aufnahmeeinrichtung (4) aufgenommenen Mikroplatte (2) mit einer anhand eines Transmissions-Bilds eines Bodens dieses Wells (3) bestimmten Konfluenz von biologischen Zellen oder Zellkulturen dieses Wells (3), und
- zum Normieren des integralen Signals eines Wells (3) mit der bestimmten Konfluenz der biologischen Zellen oder Zellkulturen in diesem Well (3).

14. Mikroplatten-Reader (1) nach Anspruch 13, **dadurch gekennzeichnet, dass** er zumindest eine Aktionsquelle (5',5'") zum Herbeiführen einer Wechselwirkung zwischen zumindest einer dieser Aktionsquellen (5',5'") und biologischen Zellen oder Zellkulturen in bestimmten Wells (3) der Mikroplatte(n) (2) und zum Hervorrufen oder Erzeugen eines messbaren Signals umfasst.

15. Mikroplatten-Reader (1) nach Anspruch 13, **dadurch gekennzeichnet, dass** er einen lichtdicht und gasdicht ausgebildeten Probenraum (12) umfasst, der als Isolierkammer ausgebildet ist,
**und dass** er ein Kontrollgerät (13) insbesondere zum Steuern der Gaszusammensetzung, der Temperatur und der relativen Feuchtigkeit in dem als Isolierkammer ausgebildeten Probenraum (12) umfasst.

16. Mikroplatten-Reader (1) nach Anspruch 14, **dadurch gekennzeichnet, dass** die zumindest eine Aktionsquelle (5',5"') ausgewählt ist aus einer Gruppe, die umfasst:
- eine Lichtquelle (5') zum Anregen einer Fluoreszenz in oder an biologischen Zellen oder Zellkulturen in Wells (3) dieser Mikroplatte(n) (2);
- eine Lichtquelle (5') zum Durchstrahlen von biologischen Zellen oder Zellkulturen in Wells (3) dieser Mikroplatte(n) (2); und
- eine Flüssigkeitsquelle (5"') zum Auslösen einer Lumineszenz in oder an biologischen Zellen oder Zellkulturen in Wells (3) dieser Mikroplatte(n) (2).

17. Mikroplatten-Reader (1) nach Anspruch 13, **dadurch gekennzeichnet, dass** die zumindest eine Messeinrichtung (6,7,8) ausgewählt ist aus einer Gruppe, die Photomultiplier, Photodioden, Photodioden-Arrays, Avalanche-Dioden und phasensensitive Lock-in-Verstärker umfasst.

18. Mikroplatten-Reader (1) nach Anspruch 13, **dadurch gekennzeichnet, dass** er eine mit der bildgebenden Kamera (10) optisch gekoppelte_mikroskopische Optik (19) umfasst.

19. Mikroplatten-Reader (1) nach Anspruch 16, **dadurch gekennzeichnet, dass** die mikroskopische Optik (19) eine Aperturblende (30) mit hoher numerischer Apertur umfasst.

20. Verwendung eines Mikroplatten-Readers (1) nach Anspruch 13 zur Durchführung des Verfahrens gemäss Anspruch 1 zum Untersuchen von biologischen Zellen oder Zellkulturen.

## Claims

1. Method for investigating biological cells or cell cultures in a microplate reader (1), wherein the method comprises:
a) providing a microplate reader (1), which comprises:
- at least one measuring device (6,7,8) having an optical axis (21', 21", 21"');
- an illumination source (9) and an imaging camera (10) with an own optical axis (20), which runs in parallel to the optical axis (21', 21", 21'") of the measuring device (6,7,8),
- a receiving device (4) for receiving at least one microplate (2) and for positioning a received microplate (2) with respect to the optical axis (21', 21", 21'") of the measuring device (6,7,8) or with respect to the optical axis (20) of the illumination source (9) and the imaging camera (10) of the microplate reader (1); and
- a processor (11');
b) positioning a well (3) of a received microplate (2) with respect to the optical axis (21', 21", 21'") of the measuring device (6,7,8) of the microplate reader (1) using the receiving device (4), and
detecting an integral signal in the well (3) of the microplate (2) using at least one of the measuring devices (6,7,8), wherein the integral signal is selected from a group comprising: a fluorescence, a luminescence, and an absorbance;
**characterized in that** the method comprises the following further steps:
c) positioning the well (3) of the microplate (2) with respect to the optical axis (20) of the illumination source (9) and the imaging camera (10) using the receiving device (4), transilluminating, and generating a transmission image of a bottom of said well (3) using the illumination source (9) and the imaging camera (10);
d) determining a confluence of biological cells or cell cultures on the transmission image of the bottom of the well (3) generated in step c),
e) comparing the detected integral signals in the well (3) of step b) with the determined confluence of the biological cells or cell cultures of step d) using the processor (11'); and
f) normalizing the integral signal of the well (3) of step b) with the confluence of the biological cells or cell cultures in said well (3) determined in step d).

2. Method according to claim 1, **characterized in that** the receiving device (4) with the biological cells or cell cultures comprising wells (3) of the microplate(s) (2) is positioned with respect to action sources (5',5'") of the microplate reader (1), and an interaction between the biological cells or cell cultures in specific wells (3) of the microplate (s) is generated using one of the action sources for bringing about or generating the integral signal.

3. Method according to claim 2, **characterized in that** the at least one action source (5', 5"') is selected from a group which comprises:
- a light source (5') for exciting a fluorescence in or on biological cells or cell cultures in wells (3) of said microplate(s) (2);
- a light source (5') for transilluminating biological cells or cell cultures in wells (3) of said microplate(s) (2); and
- a liquid source (5"') for triggering a luminescence in or on biological cells or cell cultures in wells (3) of said microplate(s) (2).

4. Method according to claim 3, **characterized in that** the light source (5') is selected from a group which comprises arc lamps, flash lamps, incandescent lamps, lasers, laser diodes, and light-emitting diodes (LEDs).

5. Method according to claim 3, **characterized in that** the liquid source (5"') is selected from a group which comprises single injectors and multiple injectors.

6. Method according to claim 1, **characterized in that** the measuring devices (6,7,8) for detecting an integral signal are selected from a group comprising photomultiplier, photodiodes, photodiode-arrays, avalanche diodes and phase-sensitive lock-in-amplifiers.

7. Method according to claim 1, **characterized in that** during positioning, the receiving device (4) with the microplate (2) comprising the biological cells or cell cultures is moved in at least one direction of movement, wherein this direction of movement is selected from a group comprising an X, a Y, and a Z direction in a three-dimensional coordinate system.

8. Method according to claim 1, **characterized in that** the wells (3) of the microplate (2) comprising the biological cells or cell cultures received by the receiving device (4) are investigated in a lightproof sample chamber (12) of the microplate reader (1).

9. Method according to claim 1, **characterized in that** the wells (3) of the microplate (2) comprising the biological cells or cell cultures received by the receiving device (4) are exposed to a controlled atmosphere in a sample chamber (12) of the microplate reader (1) which configured as an isolation chamber.

10. Method according to claim 8 or 9, **characterized in that** the received microplate (2) with the wells (3) comprising the biological cells or cell cultures is held by the receiving device (4) in the sample chamber (12) of the microplate reader (1) at least during the execution of the method steps b) and c).

11. Method according to one of the preceding claims, **characterized in that** for imaging the bottom of the wells (3) a microscope optics (19) is used which comprises an aperture diaphragm (30) with large numeric aperture.

12. Method according to claim 1, **characterized in that** all method steps a) to f) are carried out in an automated manner in a single device.

13. A microplate reader (1), which comprises
a) at least one measuring device (6,7,8) for detecting an integral signal which exists, has been brought about or produced in or on biological cells or cell cultures in a well (3) of a microplate(n) (2), wherein the measuring device (6,7,8) comprises an optical axis (21',21", 21"'), and wherein the integral signal is selected from a group which comprises: a fluorescence, a luminescence, and an absorbance;
b) an illumination source (9) and an imaging camera (10) for transilluminating a well (3) of a microplate (2), and for generating a transmission image of a bottom of said well (3), wherein the illumination source (9) and the imaging camera (10) comprise an optical axis (20) which runs in parallel to the optical axis (21', 21", 21'") of the measuring device (6,7,8), and
c) a receiving device (4) for receiving of at least one microplate (2) with wells (3) and for positioning the microplate (2) with respect to the optical axis (21', 21", 21'") of the measuring device (6,7,8) or with respect to the optical axis (20) of the illumination source (9) and the imaging camera (10);
**characterized in that** the microplate reader (1) comprises an internal processor (11'), wherein the processor (11') is configured:
- for comparing a detected integral signal of a well (3) of a microplate (2) which has been received from the receiving device (4) with a confluence of biological cells or cell cultures in said well (3) which has been determined based on a transmission image of a bottom of said well (3), and
- for normalizing the integral signal of a well (3) with the determined confluence of the biological cells or cell cultures in this well (3).

14. Microplate reader (1) according to claim 13, **characterized in that** it comprises at least one action source (5',5") for bringing about an interaction between at least one of said action sources (5',5",5"') and biological cells or cell cultures in specific wells (3) of the microplate(s) (2) and for bringing about or producing a measurable signal.

15. Microplate reader (1) according to claim 13, **characterized in that** it comprises a sample chamber (12) which is configured to be lightproof and gas-tight, and which is configured as an isolation chamber,
**and that** it comprises a control device (13) in particular for controlling the gas composition, the temperature and the relative humidity in the sample chamber (12) which is configures as an isolation chamber.

16. Microplate reader (1) according to claim 14, **characterized in that** the at least one action source (5',5"') is selected from a group which comprises:
- a light source (5') for exciting a fluorescence in or on biological cells or cell cultures in wells (3) of said microplate(s) (2);
- a light source (5') for transilluminating of biological cells or cell cultures in wells (3) of this microplate(s) (2); and
- a liquid source (5"') for triggering a luminescence in or on biological cells or cell cultures in wells (3) of this microplate(s) (2).

17. Microplate reader (1) according to claim 13, **characterized in that** the at least one measuring device (6,7,8) is selected from a group which comprises photomultiplier, photodiodes, photodiode arrays, avalanche diodes and phase-sensitive lock-in-amplifiers.

18. Microplate reader (1) according to claim 13, **characterized in that** it comprises a microscope optics (19) which is coupled optically with the imaging camera (10).

19. Microplate reader (1) according to claim 16, **characterized in that** the microscope optic (19) comprises an aperture diaphragm (30) with large numeric aperture.

20. Use of a microplate reader (1) according to claim 13 for executing the method according to claim 1 for investigating biological cells or cell cultures.

## Revendications

1. Procédé pour analyser des cellules ou cultures de cellules biologiques dans un lecteur de microplaques (1), le procédé consistant à :
a) fournir un lecteur de microplaques (1) comprenant :
- au moins un dispositif de mesure (6, 7, 8) avec un axe optique (21', 21", 21"') ;
- une source d'éclairage (9) et une caméra d'imagerie (10) disposant de son propre axe optique (20) qui s'étend parallèlement à l'axe optique (21', 21", 21'") du dispositif de mesure (6, 7, 8),
- un dispositif de réception (4) pour recevoir au moins une microplaque (2) et pour positionner une microplaque reçue (2) par rapport à l'axe optique (21', 21", 21'") du dispositif de mesure (6, 7, 8) ou par rapport à l'axe optique (20) de la source d'éclairage (9) et de la caméra d'imagerie (10) du lecteur de microplaques (1) ; et
- un processeur (11') ;
b) positionner un puits (3) d'une microplaque reçue (2) par rapport à l'axe optique (21', 21", 21'") du dispositif de mesure (6, 7, 8) du lecteur de microplaques (1) avec le dispositif de réception (4), et détecter un signal intégral dans le puits (3) de la microplaque (2) avec au moins un des dispositifs de mesure (6, 7, 8), le signal intégral étant choisi à partir d'un groupe comprenant : une fluorescence, une luminescence et une absorbance ;
**caractérisé en ce que** le procédé comprend les autres étapes suivantes:
c) positionner le puits (3) de la microplaque (2) par rapport à l'axe optique (20) de la source d'éclairage (9) et de la caméra d'imagerie (10) avec le dispositif de réception (4), radiographier et générer une image de transmission d'un fond de ce puits (3) avec la source d'éclairage (9) et la caméra d'imagerie (10) ;
d) déterminer une confluence de cellules ou de cultures de cellules biologiques sur l'image de transmission établie à l'étape c) du fond du puits (3),
e) comparer le signal intégral enregistré dans le puits (3) à l'étape b) avec la confluence déterminée des cellules ou cultures de cellules biologiques de l'étape d) à l'aide du processeur (11') ; et
f) normaliser le signal intégral du puits (3) de l'étape b) avec la confluence déterminée à l'étape d) des cellules ou cultures de cellules biologiques dans ce puits (3).

2. Procédé selon la revendication 1, **caractérisé en ce que** le dispositif de réception (4) est positionné avec les puits (3) de la ou des microplaques (2) contenant les cellules ou cultures de cellules biologiques par rapport aux sources d'action (5', 5'") du lecteur de microplaques (1), et **en ce qu'**avec une des sources d'action il est produit une interaction avec les cellules ou cultures de cellules biologiques dans certains puits (3) de la ou des microplaques (2) pour provoquer ou produire le signal intégral.

3. Procédé selon la revendication 2, **caractérisé en ce que** la au moins une source d'action (5', 5'") est choisie à partir d'un groupe comprenant :
- une source lumineuse (5') pour stimuler une fluorescence dans ou au niveau des cellules ou cultures de cellules biologiques dans les puits (3) de cette ou ces microplaques (2) ;
- une source lumineuse (5') pour radiographier les cellules ou cultures de cellules biologiques dans les puits (3) de cette ou ces microplaques (2) ;
- une source de liquide (5'") pour déclencher une luminescence dans ou au niveau des cellules ou cultures de cellules biologiques dans les puits (3) de cette ou ces microplaques (2).

4. Procédé selon la revendication 3, **caractérisé en ce que** la source lumineuse (5') est choisie à partir d'un groupe comprenant des lampes à arc, des lampes flash, des ampoules à incandescence, des lasers, des diodes laser et des diodes électroluminescentes (LED).

5. Procédé selon la revendication 3, **caractérisé en ce que** la source de liquide (5"') est choisie à partir d'un groupe comprenant des injecteurs simples et des injecteurs multiples.

6. Procédé selon la revendication 1, **caractérisé en ce que** les dispositifs de mesure (6, 7, 8) pour détecter un signal intégral sont choisis à partir d'un groupe comprenant des photomultiplicateurs, des photodiodes, des réseaux de photodiodes, des diodes à avalanche et des amplificateurs à verrouillage sensibles en phase.

7. Procédé selon la revendication 1, **caractérisé en ce que** le dispositif de réception (4) est déplacé, avec la microplaque (2) contenant les cellules ou cultures de cellules biologiques, lors du positionnement, dans au moins un sens de déplacement, ce sens de déplacement étant choisi à partir d'un groupe comprenant une direction X, une direction Y et une direction Z dans un système de coordonnées en trois dimensions.

8. Procédé selon la revendication 1, **caractérisé en ce que** les puits (3) de la microplaque (2) contenant les cellules ou cultures de cellules biologiques reçues par le dispositif de réception (4) sont analysés dans un compartiment à échantillon (12) étanche à la lumière du lecteur de microplaques (1).

9. Procédé selon la revendication 1, **caractérisé en ce que** les puits (3) de la microplaque (2) contenant les cellules ou cultures de cellules biologiques reçues par le dispositif de réception (4) sont installés dans un compartiment à échantillon (12) réalisé sous la forme d'un compartiment isolant du lecteur de microplaques (1) sous une atmosphère contrôlée.

10. Procédé selon la revendication 8 ou 9, **caractérisé en ce que** la microplaque reçue (2) avec les puits (3) contenant les cellules ou cultures de cellules biologiques sont maintenus dans le compartiment à échantillon (12) du lecteur de microplaques (1) par le dispositif de réception (4) au moins pendant la réalisation des étapes de procédé b) et c).

11. Procédé selon l'une des revendications précédentes, **caractérisé en ce que** pour reproduire le fond du puits (3) on utilise une optique microscopique (19) qui comprend un diaphragme (30) avec une ouverture numérique élevée.

12. Procédé selon la revendication 1, **caractérisé en ce que** toutes les étapes a) à f) sont réalisées de façon automatisée dans un seul appareil.

13. Lecteur de microplaques (1) comprenant :
a) au moins un dispositif de mesure (6, 7, 8) pour détecter un signal intégral qui existe, a été provoqué ou produit dans ou au niveau de cellules ou cultures de cellules biologiques dans un puits (3) d'une microplaque (2), le dispositif de mesure (6, 7, 8) comprenant un axe optique (21', 21", 21"'), et le signal intégral étant choisi à partir d'un groupe comprenant : une fluorescence, une luminescence et une absorbance ;
b) une source d'éclairage (9) et une caméra d'imagerie (10) pour radiographier un puits (3) d'une microplaque (2) et pour établir une image de transmission d'un fond d'un puits (3), la source d'éclairage (9) et la caméra d'imagerie (10) comprenant un axe optique (20) qui s'étend parallèlement à l'axe optique (21', 21", 21'") du dispositif de mesure (6, 7, 8), et
c) un dispositif de réception (4) pour recevoir au moins une microplaque (2) avec des puits (3) et pour positionner la microplaque (2) par rapport à l'axe optique (21', 21", 21"') du dispositif de mesure (6, 7, 8) ou par rapport à l'axe optique (20) de la source d'éclairage (9) et de la caméra d'imagerie (10) ;
**caractérisé en ce que** le lecteur de microplaques (1) comprend un processeur interne (11'), le processeur (11') étant conçu :
- pour comparer un signal intégral enregistré d'un puits (3) d'une microplaque (2) reçue par le dispositif de réception (4) avec une confluence de cellules ou de cultures de cellules biologiques de ce puits (3) déterminée à l'aide d'une image de transmission d'un fond de ce puits (3), et
- pour normaliser le signal intégral d'un puits (3) avec la confluence déterminée des cellules ou cultures de cellules biologiques dans ce puits (3).

14. Lecteur de microplaques (1) selon la revendication 13, **caractérisé en ce qu'**il comprend au moins une source d'action (5', 5'") pour produire une interaction entre au moins une de ces sources d'action (5', 5"') et les cellules ou cultures de cellules biologiques dans certains puits (3) de la ou des microplaques (2) et pour provoquer ou produire un signal mesurable.

15. Lecteur de microplaques (1) selon la revendication 13, **caractérisé en ce qu'**il comprend un compartiment à échantillon (12) conçu étanche à la lumière et au gaz, qui est conçu comme un compartiment isolant,
**et en ce qu'**il comprend un appareil de contrôle (13) en particulier pour contrôler la composition du gaz, la température et l'humidité relative dans le compartiment à échantillon (2) conçu comme un compartiment isolant.

16. Lecteur de microplaques (1) selon la revendication 14, **caractérisé en ce que** la au moins une source d'action (5', 5"') est choisie à partir d'un groupe comprenant :
- une source lumineuse (5') pour déclencher une fluorescence dans ou au niveau des cellules ou cultures de cellules biologiques dans les puits (3) de cette ou ces microplaques (2) ;
- une source lumineuse (5') pour radiographier les cellules ou cultures de cellules biologiques dans les puits (3) de cette ou ces microplaques (2) ; et
- une source de liquide (5'") pour déclencher une luminescence dans ou au niveau des cellules ou cultures de cellules biologiques dans les puits (3) de cette ou ces microplaques (2).

17. Lecteur de microplaques (1) selon la revendication 13, **caractérisé en ce que** le au moins un dispositif de mesure (6, 7, 8) est choisi à partir d'un groupe comprenant des photomultiplicateurs, des photodiodes, des réseaux de photodiodes, des diodes à avalanche et des amplificateurs à verrouillage sensibles en phase.

18. Lecteur de microplaques (1) selon la revendication 13, **caractérisé en ce qu'**il comprend une optique microscopique (19) couplée optiquement à la caméra d'imagerie (10).

19. Lecteur de microplaques (1) selon la revendication 16, **caractérisé en ce que** l'optique microscopique (19) comprend un diaphragme (30) avec une ouverture numérique élevée.

20. Utilisation d'un lecteur de microplaques (1) selon la revendication 13 pour effectuer le procédé selon la revendication 1 pour analyser des cellules ou cultures de cellules biologiques.
